# EUROPEAN PATENT APPLICATION

(11) **EP 4 831 168 A1**
(43) Date of publication of application: **28.10.2026**
(21) Application number: 24906506.1
(22) Date of filing: 20.12.2024
(51) Int. Cl.: C07D 403/12, C07D 249/08

(54) **TYK2 INHIBITOR AND PREPARATION METHOD FOR INTERMEDIATES THEREOF**

(30) Priority: 22.12.2023 CN 202311773442
(71) Applicant: Shanghai Aobo Pharmtech, Inc., Ltd., Shanghai 201203 (CN); Zhejiang Huahai Pharmaceutical Co., Ltd., Taizhou, Zhejiang 317024 (CN)
(72) Inventor: WANG, Quan, Taizhou, Zhejiang 317024 (CN); ZHANG, Jicheng, Taizhou, Zhejiang 317024 (CN); GAO, Beibei, Taizhou, Zhejiang 317024 (CN); GU, Zhengjie, Taizhou, Zhejiang 317024 (CN); HUANG, Luning, Taizhou, Zhejiang 317024 (CN); TAO, Anping, Taizhou, Zhejiang 317024 (CN); GU, Hong, Taizhou, Zhejiang 317024 (CN)
(74) Representative: Pfenning, Meinig & Partner mbB
(86) International application number: PCT/CN2024/140851
(87) International publication number: WO 2025/131031

(57) **Abstract**

The present invention provides a TyK2 inhibitor and a preparation method for intermediates thereof, and particularly relates to preparation for a compound I and an intermediate compound IV and compound V thereof. The compound I and the intermediates thereof prepared by means of the method involve a mild reaction, simple post-treatment, high purity and yield.

## Description

The present application claims the priority of Chinese Patent Application No. CN202311773442.5 filed before the CNIPA on December 22, 2023, which is incorporated herein by reference in its entirety.

### TECHNICAL FIELD

The present application belongs to the technical field of medicine, and particularly relates to the preparation of 6-(cyclopropanecarboxamido)-4-((2-methoxy-3-(1-methyl-1H-1,2,4-triazol-3-yl)phen yl)amino)-N-(methyl-d3)pyridazine-3-carboxamide and intermediates thereof.

### BACKGROUND

The compound 6-(cyclopropanecarboxamido)-4-((2-methoxy-3-(1-methyl-1*H*-1,2,4-triazol-3-yl)phen yl)amino)-*N*-(methyl-*d*₃)pyridazine-3-carboxamide (hereinafter referred to as "compound I") has the following structural formula:

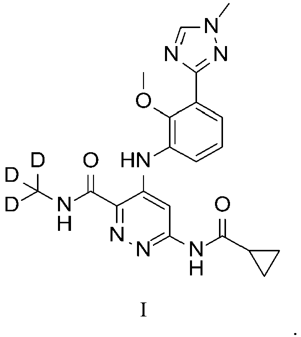

Compound I is a TyK2 inhibitor for use in the treatment of autoimmune diseases and autoinflammatory diseases. Different from known JAK inhibitors, compound I selectively binds to the pseudokinase domain (JH2) of TyK2, inhibiting TyK2 kinase activity through a molecular allosteric mechanism, and has weak inhibitory activity against JAK1-3, effectively reducing adverse reactions caused by inhibition of JAK kinases 1-3.

WO2023102085A1 discloses a preparation method of compound I: (1) 2-methoxy-3-(1-methyl-1H-1,2,4-triazol-3-yl)aniline (compound III) and ethyl 4,6-dichloropyridazine-3-carboxylate (compound IIa) in the presence of 2,2,6,6-tetramethylpiperidine in toluene were stirred at 110 °C until completion of the reaction. T, Then, the reaction solution was cooled to about 20 °C, toluene and water were added and stirred for 1 hour, then filtered. The filter cake was washed twice with toluene, then twice with water, and dried under reduced pressure at about 50 °C to obtain a solid: ethyl 6-chloro-4-((2-methoxy-3-(1-methyl-1H-1,2,4-triazol-3-yl)phenyl)amino)pyridazine-3-carboxylate (compound IVa), yield 80%. (2) Ethyl 6-chloro-4-((2-methoxy-3-(1-methyl-1H-1,2,4-triazol-3-yl)phenyl)amino)pyridazine-3-carboxylate (compound IVa) in the presence of lithium bis(trimethylsilyl)amide was reacted with trideuteromethylamine hydrochloride in tetrahydrofuran at room temperature. After completion of the reaction, the reaction was quenched by slow addition of 2:1 (v/v) water/saturated aqueous ammonium chloride solution, the layers were separated, and the upper layer was evaporated to dryness to obtain a solid compound:
6-chloro-4-((2-methoxy-3-(1-methyl-1H-1,2,4-triazol-3-yl)phenyl)amino)-N-(methyl-d3)pyridazine-3-carboxamide (compound Va), yield 75%. (3) 6-Chloro-4-((2-methoxy-3-(1-methyl-1H-1,2,4-triazol-3-yl)phenyl)amino)-N-(methyl -d3)pyridazine-3-carboxamide (compound Va) was reacted with cyclopropanecarboxamide in the presence of potassium carbonate, palladium acetate and a Josiphos ligand in acetonitrile and toluene at about 75 °C. The reaction mixture was cooled to about 20 °C and quenched by slow addition of 3:1 (v/v) water/saturated aqueous ammonium chloride solution, then filtered. The filter cake was washed twice with water, and the solid was dried under reduced pressure at about 80 °C to obtain compound I in yield 80%.

In the above preparation method, the first step reaction is carried out in toluene at 110 °C, which is a relatively high reaction temperature. The second step reaction has very strict requirements for controlling the water content in the reaction system. When the reaction system contains water, the following impurity 1 is generated, which will hinder the transesterification of the substrate to form the corresponding product. The third step reaction uses a mixed solvent, which is not conducive to solvent recovery and reuse. Additionally, the post-treatment procedures for the above three steps are all relatively complicated and generate a large amount of waste water.

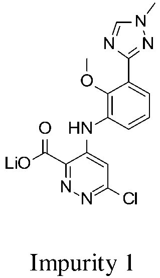

### SUMMARY

A first aspect of the present application provides a preparation method of compound V:
mixing deuterated methylamine hydrochloride with an inorganic base in a solvent, and then adding compound IV and an organic base to react to form compound V, as represented by the following reaction: 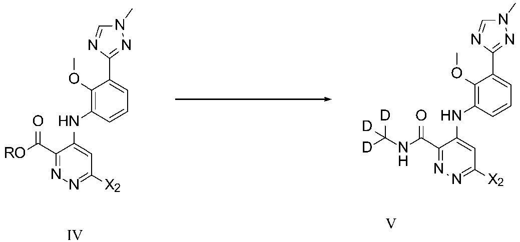
wherein R is C₁₋₆ alkyl or aryl, X₂ is selected from the group consisting of Cl, Br, I and sulfonate group, and the organic base is a metal ion-free non-nucleophilic organic base.

In some embodiments, the preparation of compound V is carried out in a sealed container.

In some embodiments, R is selected from the group consisting of methyl and ethyl, and X₂ is Cl.

In some embodiments, compound IV is compound IVa, compound V is compound Va, and compound IVa and compound Va are respectively as follows:

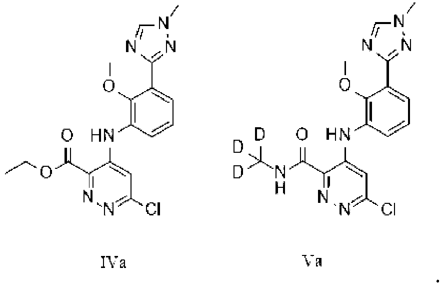

In some embodiments, the inorganic base is optionally first dissolved in water to prepare an aqueous solution containing the inorganic base.

In some embodiments, the inorganic base is selected from one or more of LiOH, NaOH, KOH, Ca(OH)₂, Ba(OH)₂, Na₂CO₃, K₂CO₃, NaHCO₃, KHCO₃, NaNH₂ and NH₃ ·H₂O. In some embodiments, the inorganic base is selected from the group consisting of NaOH and KOH.

In some embodiments, the inorganic base is 50% (w/w) sodium hydroxide solution, which is prepared by mixing equal masses of water and sodium hydroxide.

In some embodiments, a molar ratio of deuterated methylamine hydrochloride to the inorganic base is 1:1 to 1.5.

In some embodiments, the molar ratio of deuterated methylamine hydrochloride to the inorganic base is 1:1 to 1.2.

In some embodiments, the metal ion-free non-nucleophilic organic base is selected from one or more of DIEA, DBU, DBN, DMAP, 2,6-di-tert-butylpyridine, urea and thiourea. In some embodiments, the metal ion-free non-nucleophilic organic base is selected from one or more of DBU, DIEA, DMAP, urea and thiourea.

In some embodiments, the molar ratio of compound IV to the metal ion-free non-nucleophilic organic base is 1:0.001 to 0.5. In some embodiments, the molar ratio of compound IV to the metal ion-free non-nucleophilic organic base is 1:0.01 to 0.5. In some embodiments, the molar ratio of compound IV to the metal ion-free non-nucleophilic organic base is 1:0.01 to 0.3. In some embodiments, the molar ratio of compound IV to the metal ion-free non-nucleophilic organic base is 1:0.05 to 0.15.

In some embodiments, the solvent is selected from one or more of THF, DMF, methanol, ethanol, propanol, isopropanol, 1,4-dioxane and water. In some embodiments, the solvent is selected from one or more of THF, ethanol and water. In some embodiments, the solvent is ethanol. In some embodiments, the solvent is THF. In some embodiments, the solvent is a mixed solvent of THF and water.

In some embodiments, a mass-to-volume ratio of compound IV to the solvent is 1:5 to 20. In some embodiments, a mass-to-volume ratio of compound IV to the solvent is 1:10 to 15.

In some embodiments, a molar ratio of compound IV to deuterated methylamine hydrochloride is 1:1 to 1.5. In some embodiments, the molar ratio of compound IV to deuterated methylamine hydrochloride is 1:1 to 1.3.

In some embodiments, a temperature for a reaction of compound IV with deuterated methylamine hydrochloride is 20 °C to 40 °C.

In some embodiments, a temperature for a reaction of compound IV with deuterated methylamine hydrochloride is 20 °C to 35 °C.

In some preferred embodiments, the preparation method of compound V further comprises the preparation of compound IV, and the preparation method of compound IV comprises the following step: reacting compound II with compound III to obtain compound IV, as represented by the following reaction: 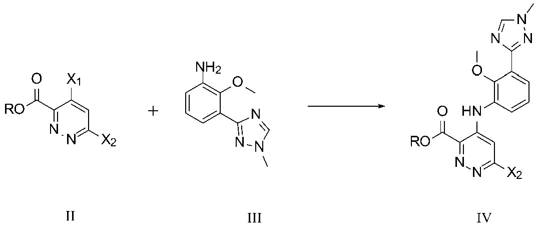 wherein R is C₁₋₆ alkyl or aryl, and X₁ and X₂ are independently selected from the group consisting of Cl, Br, I and sulfonate group.

In some embodiments, compound II is reacted with compound III in a protic solvent in the presence of an organic base to form compound IV, and the organic base is a metal ion-free non-nucleophilic organic base.

In some embodiments, R is selected from the group consisting of methyl and ethyl, and X₁ and X₂ are Cl.

In some embodiments, compound II is compound IIa, compound IV is compound IVa, and compound IIa and compound IVa are respectively as follows:

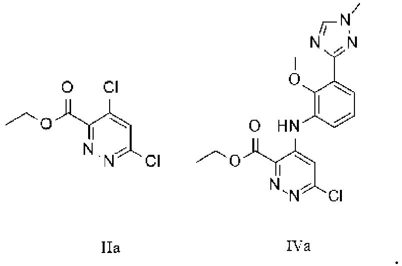

In some embodiments, the protic solvent is an aliphatic alcohol containing 1 to 6 carbon atoms.

In some embodiments, the protic solvent is selected from the group consisting of methanol, ethanol and isopropanol.

In some embodiments, the protic solvent is ethanol.

In some embodiments, the metal ion-free non-nucleophilic organic base is selected from one or more of DIEA, DBU, DBN, DMAP, 2,6-di-tert-butylpyridine, urea and thiourea. In some embodiments, the metal ion-free non-nucleophilic organic base is selected from one or more of DBU, DIEA, DMAP, urea and thiourea. In some embodiments, the metal ion-free non-nucleophilic organic base is selected from the group consisting of DBU and DIEA.

In some embodiments, a molar ratio of compound III to compound II is 1:1 to 1.5. In some embodiments, the molar ratio of compound III to compound II is 1:1 to 1.3.

In some embodiments, the molar ratio of compound III to the metal ion-free non-nucleophilic organic base is 1:1 to 3. In some embodiments, the molar ratio of compound III to the metal ion-free non-nucleophilic organic base is 1:2 to 3.

In some embodiments, a mass-to-volume ratio of compound III to the protic solvent is 1:5 to 20.

In some embodiments, the mass-to-volume ratio of compound III to the protic solvent is 1:8 to 15.

In some embodiments, a temperature for a reaction of compound II with compound III is 60 °C to 85 °C.

In some embodiments, a temperature for the reaction of compound II with compound III is 65 °C to 80 °C. In some preferred embodiments, the preparation method of compound V uses a one-pot process starting from compound II and compound III, specifically comprising the following steps: reacting compound II with compound III in a protic solvent in the presence of a metal ion-free non-nucleophilic organic base; adding additional identical base after completion of the reaction, then adding deuterated methylamine or a salt thereof to continue the reaction to form compound V.

Wherein, the protic solvent, the metal ion-free non-nucleophilic organic base, the reaction ratios and conditions are as described above.

In some preferred embodiments, when the preparation method of compound V uses the above one-pot process starting from compound II and compound III, the molar ratio of deuterated methylamine or a salt thereof to compound IV, and the molar ratio of the additional base to compound IV are both based on the theoretical molar ratios relative to compound IV, with the ratios as described above.

By preparing compound V or compound Va via the above method, the amount of non-nucleophilic organic base used is greatly reduced, lowering the cost. Moreover, because the non-nucleophilic organic base used contains no metal ions, compared with LiHMDS, it has low requirements for water content in the reaction system. The above preparation method can be carried out in an aqueous mixed solvent, with mild reaction conditions, less generation of impurities, and simple post-treatment. After the reaction is complete, there is no need to add aqueous ammonium chloride solution for quenching. The solid compound V or compound Va can be directly obtained by cooling and crystallization, with high purity and yield.

A second aspect of the present application provides a preparation method of compound I:
reacting compound V with cyclopropanecarboxamide in a non-polar organic solvent in the presence of a base, a palladium catalyst and a phosphine ligand, as represented by the following reaction: 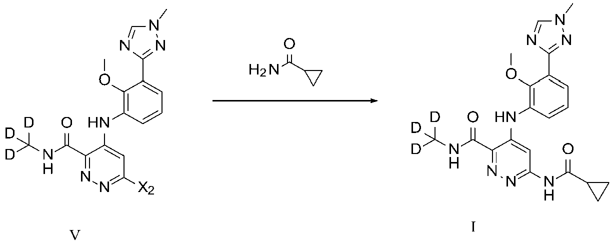
wherein X₂ is independently selected from the group consisting of Cl, Br, I and sulfonate group.

In some embodiments, X₂ is Cl.

In some embodiments, the base is selected from the group consisting of an inorganic base, an organic base and a mixture thereof.

In some embodiments, the base is a mixture of an inorganic base and an organic base.

In some embodiments, the non-polar solvent is a single solvent.

In some embodiments, the inorganic base is selected from one or more of LiOH, NaOH, KOH, Ca(OH)₂, Ba(OH)₂ , Na₂CO₃, K₂CO₃, NaHCO₃, KHCO₃, NaNH₂, NH₃ ·H₂O, K₃PO₃ and K₂HPO₃. In some embodiments, the inorganic base is selected from the group consisting of K₂CO₃ and K₃PO₃.

In some embodiments, the molar ratio of compound V to the inorganic base is 1:1 to 3. In some embodiments, the molar ratio of compound V to the inorganic base is 1:1.5 to 2.5.

In some embodiments, the organic base is a metal ion-free non-nucleophilic organic base. In some embodiments, the metal ion-free non-nucleophilic organic base is selected from one or more of DIEA, DBU, DBN, DMAP, 2,6-di-tert-butylpyridine, urea and thiourea. In some embodiments, the metal ion-free non-nucleophilic organic base is selected from one or more of DBU, DIEA, DMAP, urea and thiourea. In some embodiments, the metal ion-free non-nucleophilic organic base is selected from the group consisting of DBU and DIEA.

In some embodiments, the molar ratio of compound V to the metal ion-free non-nucleophilic organic base is 1:1 to 5. In some embodiments, the molar ratio of compound V to the metal ion-free non-nucleophilic organic base is 1:2 to 3.

In some embodiments, the palladium catalyst is selected from the group consisting of Pd(OAc)_{2,} PdCl₂(MeCN)₂, Pd₂(dba)₃, Pd(dba)₂, [(allyl)PdCl]₂ and [(crotyl)PdCl]₂. In some embodiments, the palladium catalyst is Pd(OAc)₂. In some embodiments, the molar ratio of compound V to the palladium catalyst is 1:0.005 to 0.1. In some embodiments, the molar ratio of compound V to the palladium catalyst is 1:0.01 to 0.05.

In some embodiments, the phosphine ligand is selected from the group consisting of a Josiphos ligand and a Xantphos ligand. In some embodiments, the Josiphos ligand can be (R)-N,N-dimethyl-1-((S)-2-diphenylphosphino)ferrocenyl)ethylamine or (R)-(-)-1-[(S)-2-(dicyclohexylphosphino)ferrocenyl]ethyldi-tert-butylphosphine. In some embodiments, the Xantphos ligand is 4,5-bis(diphenylphosphino)-9,9-dimethylxanthene. In some embodiments, the molar ratio of compound V to the phosphine ligand is 1:0.01 to 0.1. In some embodiments, the molar ratio of compound V to the phosphine ligand is 1:0.02 to 0.05.

In some embodiments, the molar ratio of compound V to cyclopropanecarboxamide is 1:1 to 5. In some embodiments, the molar ratio of compound V to cyclopropanecarboxamide is 1:1 to 4. In some embodiments, the molar ratio of compound V to cyclopropanecarboxamide is 1:1 to 3. In some embodiments, the molar ratio of compound V to cyclopropanecarboxamide is 1:1 to 2. In some embodiments, the molar ratio of compound V to cyclopropanecarboxamide is 1:1 to 1.5. In some embodiments, the molar ratio of compound V to cyclopropanecarboxamide is 1:1 to 1.3.

In some embodiments, the non-polar organic solvent is an ether solvent, an aromatic solvent, or an ester solvent. In some embodiments, the non-polar organic solvent is selected from the group consisting of DME and toluene. In some embodiments, the mass-to-volume ratio of compound V to the non-polar organic solvent is 1:5 to 20. In some embodiments, the mass-to-volume ratio of compound V to the non-polar organic solvent is 1:10 to 15. In some embodiments, a temperature for the reaction is 70 °C to 110 °C.

In some embodiments, the preparation of compound I further comprises the preparation of compound V; in some embodiments, the preparation method of compound V is as described in the first aspect of the present application.

A third aspect of the present application provides a preparation method of compound IV, comprising the following step:
reacting compound II with compound III in a protic solvent in the presence of an organic base to form compound IV, as represented by the following reaction: 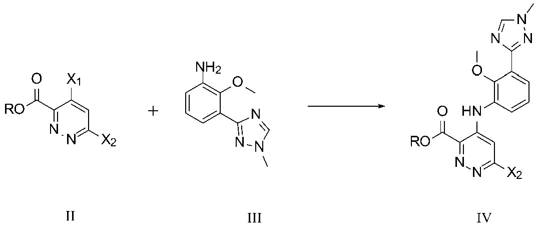
wherein R is C₁₋₆ alkyl or aryl, X₁ and X₂ are independently selected from the group consisting of Cl, Br, I and sulfonate group, and the organic base is a metal ion-free non-nucleophilic organic base.

In some embodiments, R is selected from the group consisting of methyl and ethyl, and X₁ and X₂ are Cl.

In some embodiments, compound II is compound IIa, compound IV is compound IVa, and compound IIa and compound IVa are respectively as follows:

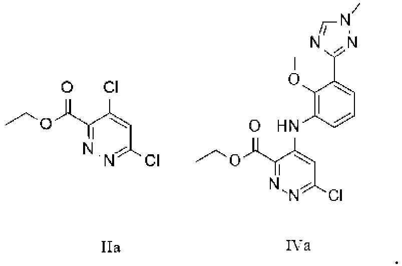

In some embodiments, the selection of the organic base and protic solvent, the amounts and ratios of each component, the reaction temperature, etc., involved in the preparation of compound IV or compound IVa are as described in the preparation method of compound IV or compound IVa in the first aspect of the present application.

By preparing compound IV or compound IVa via the above method, the reaction conditions are mild, the post-treatment is simple, and there is no need to add water for washing. The solid compound IV or compound IVa can be directly obtained by cooling and crystallization, with high purity and yield. Moreover, the solvent used is a single alcohol-type solvent, which is easy to recover and reuse, produces no waste water, and is environmentally friendly.

A fourth aspect of the present application provides a preparation method of compound I, 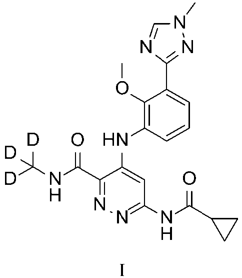 comprising the following steps:
(1) preparing compound IVa
   reacting compound IIa with compound III in an alcohol solvent in the presence of an organic base to form compound IVa, as represented by the following reaction: 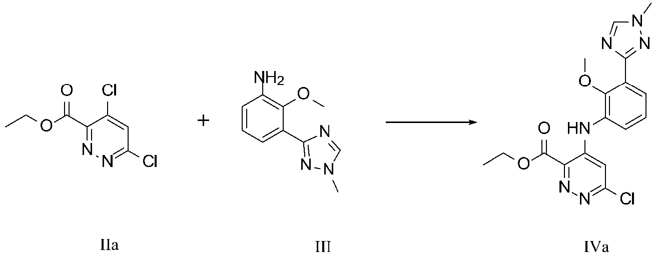
(2) preparing compound Va
   mixing deuterated methylamine hydrochloride with an inorganic base in a solvent, then adding compound IVa and an organic base to react to form compound Va, as represented by the following reaction: 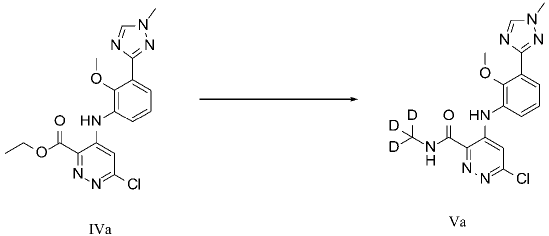
(3) preparing compound I
   reacting compound Va with cyclopropanecarboxamide in a non-polar organic solvent in the presence of a base, a palladium catalyst and a phosphine ligand to form compound I, as represented by the following reaction:

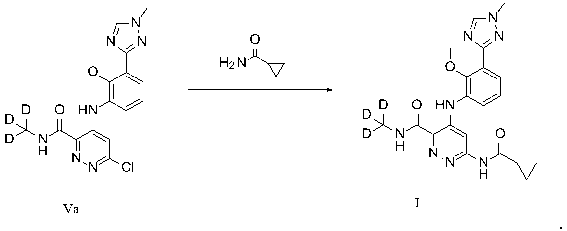

In some embodiments, the selection of the organic base, the amounts and ratios of each component, the reaction temperature, etc., involved in the preparation of compound IVa in step (1) are as described in the preparation method of compound IV or compound IVa in the first aspect of the present application.

In some embodiments, the alcohol solvent in step (1) is selected from the group consisting of methanol, ethanol and isopropanol.

In some embodiments, the mass-to-volume ratio of compound III to the alcohol solvent in step (1) is 1:5 to 20.

In some embodiments, the mass-to-volume ratio of compound III to the alcohol solvent in step (1) is 1:8 to 15.

In some embodiments, the preparation of compound Va in step (2) is carried out in a sealed container, and the selection of the inorganic base, organic base and solvent, the amounts and ratios of each component, the reaction temperature, etc., are as described in the preparation method of compound V or compound Va in the first aspect of the present application.

In some embodiments, the organic bases in steps (1) and (2) are both metal ion-free non-nucleophilic organic bases; in some embodiments, the organic bases in steps (1) and (2) are identical.

In some embodiments, the selection of the inorganic base, organic base, palladium catalyst, phosphine ligand and non-polar organic solvent, the amounts and ratios of each component, the reaction temperature, etc., involved in the preparation of compound I in step (3) are as described in the preparation method of compound I in the second aspect of the present application.

In some embodiments, after completion of the reaction in step (1), step (2) and step (3), the corresponding solid compounds IVa, Va, and I can be obtained by cooling and crystallization. The obtained solid compounds can be optionally further purified by conventional methods such as recrystallization.

In some embodiments, the present application further provides methods for preparing compound I starting from compound IVa or compound Va, with the reactions as described above.

Compound I prepared by the above preparation methods has mild reaction conditions, simple post-treatment, and high purity and yield.

### DETAILED DESCRIPTION

The following specific examples are further detailed descriptions of the present application, and the examples are for illustration only and should not be considered as limiting the present application in any way.

In the present application, unless otherwise specified, scientific and technical terms used herein have the meanings commonly understood by those skilled in the art.

In the present application, "C₁₋₆ alkyl" refers to a branched or straight-chain saturated aliphatic hydrocarbon group having a specified number of carbon atoms, including, but not limited to, methyl, ethyl, propyl (e.g., n-propyl and isopropyl), butyl (e.g., n-butyl, isobutyl, tert-butyl), pentyl (e.g., n-pentyl, isopentyl, neopentyl), and the like.

In the present application, "aryl" refers to a monocyclic or bicyclic aromatic hydrocarbon group having 6 to 12 carbon atoms in the ring, such as phenyl and naphthyl, each of which may be substituted.

In the present application, "sulfonate group" refers to a group having the general structure -OSO₂R₃, where R₃ can optionally be alkyl or aryl. The sulfonate group includes, but is not limited to, mesyloxy, ethylsulfonyloxy, tosyloxy.

The compounds II and III used in the present application can be purchased or prepared by methods disclosed in existing literature such as CN110475774. Unless otherwise specified, the mass-to-volume ratio in the present application is in g/mL.

The abbreviations used in the present application are explained as follows:
DIEA: N,N-diisopropylethylamine
DBU: 1,8-diazabicyclo[5.4.0]undec-7-ene
DMAP: dimethylaminopyridine
DBN: 9,9-dimethylxanthene
DME: dimethoxyethane
LiHMDS: lithium bis(trimethylsilyl)amide
IPA: isopropanol
THF: tetrahydrofuran
w/w: mass/mass ratio

### Specific examples are as follows:

### Comparative Example 1: Preparation of compound IVa, carried out according to the method of Example 28 of WO2023102085A1

1.98 mL of 2,2,6,6-tetramethylpiperidine, 2.60 g of compound IIa and 2 g of compound III were added to 11 mL of toluene, stirred at 110 °C until completion of the reaction. The reaction solution was cooled to 20 °C, toluene and water were added to quench the reaction. After stirring for 1 h, the mixture was filtered, and the filter cake was rinsed with toluene and water, and dried at 50 °C to obtain 2.92 g of solid compound IVa. Yield 76.7%, purity 98.27%.

### Comparative Example 2: Preparation of compound IVa

2.60 g of compound IIa, 2 g of compound III and 3.16 g of DIEA were added to 20 mL of toluene. The reaction temperature was raised to 110 °C, and the reaction solution was stirred at that temperature until completion of the reaction, then cooled to 25 °C to 35 °C. 30 mL of water was added to the system, the pH was adjusted to 6 to 7, and the filter cake was rinsed with a small amount of toluene and dried to obtain 2.81 g of solid compound IVa. Yield 73.8%, purity 96.14%.

### Example 1: Preparation of compound IVa

90.91 g of compound IIa, 70 g of compound III and 110.75 g of DIEA were added to 700 mL of ethanol. The reaction temperature was raised to 78 °C, and the reaction solution was stirred at that temperature until completion of the reaction, then cooled to 25 °C to 35 °C, crystallized by cooling, and filtered. The filter cake was rinsed with a small amount of ethanol and dried to obtain 107.01 g of solid compound IVa. Yield 80.3%, purity 99.49%.

### Example 2: Preparation of compound IVa

11.36 g of compound IIa, 10 g of compound III and 15.82 g of DIEA were added to 100 mL of IPA. The reaction temperature was raised to 80 °C, and the reaction solution was stirred at that temperature until completion of the reaction, then cooled to 0 °C to 5 °C, crystallized by cooling, and filtered. The filter cake was rinsed with a small amount of IPA and dried to obtain 16.03 g of solid compound IVa. Yield 84.2%, purity 97.39%.

### Example 3: Preparation of compound IVa

11.36 g of compound IIa, 10 g of compound III and 15.82 g of DIEA were added to 100 mL of methanol. The reaction temperature was raised to reflux temperature, and the reaction solution was stirred at that temperature until completion of the reaction, then cooled to 25 °C to 35 °C, crystallized by cooling, and filtered. The filter cake was rinsed with a small amount of methanol and dried to obtain 15.88 g of solid compound IVa. Yield 83.4%, purity 99.27%.

### Example 4: Preparation of compound IVa

11.91 g of compound IIa, 10 g of compound III and 18.64 g of DBU were added to 100 mL of ethanol. The reaction temperature was raised to 80 °C, and the reaction solution was stirred at that temperature until completion of the reaction. The resultant was filtered. The filter cake was rinsed with ethanol and dried to obtain 15.42 g of compound IVa. Yield 81.0%, purity 98.85%.

### Comparative Example 3: Preparation of compound Va

The reaction was carried out according to the method of Example 31 of WO2023102085A1.

5.6 mL of LiHMDS, 2 g of compound IVa, and 0.408 g of deuterated methylamine hydrochloride were added to 20 mL of THF. The mixture was reacted at 20 °C until completion. Then, 30 mL of a mixed solvent of 2:1 (v/v) water/saturated aqueous ammonium chloride solution was slowly added to quench the reaction. The layers were separated, and the upper organic phase was evaporated to dryness to obtain 1.43 g of solid compound Va. Yield 73.8%, purity 98.07%.

### Example 5: Preparation of compound Va

14 g of deuterated methylamine hydrochloride and 10 mL of 50% (w/w) NaOH solution were added to 700 mL of THF. The mixture was stirred under sealed conditions at 0 °C to 10 °C for 2 h. Then, 70 g of compound IVa and 2.57 g of DBU were added, and the reaction was carried out under sealed conditions at 30 °C. After completion, 2100 mL of water was added, stirred for 0.5 h. THF was removed by distillation under reduced pressure, and the mixture was filtered and dried to obtain 67 g of solid compound Va. Yield 98.8%, purity 99.10%.

### Example 6: Preparation of compound Va

0.40 g of deuterated methylamine hydrochloride and 0.32 g of KOH were added to 20 mL of ethanol. The mixture was stirred under sealed conditions at 0 °C to 10 °C for 1 h. Then, 2 g of compound IVa and 0.07 g of DIEA were added, and the reaction was carried out under sealed conditions at 30 °C. After completion, 60 mL of water was added, stirred for 0.5 h, and the mixture was filtered and dried to obtain 1.81 g of solid compound Va. Yield 93.4%, purity 95.17%.

### Example 7: Preparation of compound Va

0.40 g of deuterated methylamine hydrochloride and 0.30 mL of 50% (w/w) NaOH solution were added to 20 mL of THF. The mixture was stirred under sealed conditions at 0 °C to 10 °C for 1 h. Then 2 g of compound IVa and 0.06 g of DMAP were added, and the reaction was carried out under sealed conditions at 30 °C. After completion, 60 mL of water was added, stirred for 0.5 h. THF was removed by distillation under reduced pressure, and the mixture was filtered and dried to obtain 1.80 g of solid compound Va. Yield 92.8%, purity 95.85%.

### Example 8: Preparation of compound Va

0.40 g of deuterated methylamine hydrochloride and 0.28 mL of 50% (w/w) NaOH solution were added to 20 mL of THF. The mixture was stirred under sealed conditions at 0 °C to 10 °C for 2 h. Then, 2 g of compound IVa and 0.03 g of urea were added, and the reaction was carried out under sealed conditions at 35 °C. After completion, 60 mL of water was added, stirred for 0.5 h. THF was removed by distillation under reduced pressure, and the mixture was filtered and dried to obtain 1.79 g of solid compound Va. Yield 92.3%, purity 95.94%.

### Example 9: Preparation of compound Va

0.44 g of deuterated methylamine hydrochloride and 0.30 mL of 50% (w/w) NaOH solution were added to 30 mL of THF. The mixture was stirred under sealed conditions at 0 °C to 10 °C for 2 h. Then, 2 g of compound IVa and 0.06 g of thiourea were added, and the reaction was carried out under sealed conditions at 30 °C. After completion, 80 mL of water was added, stirred for 0.5 h. THF was removed by distillation under reduced pressure, and the mixture was filtered and dried to obtain 1.73 g of solid compound Va. Yield 89.2%, purity 96.99%.

### Comparative Example 4: Preparation of compound I.

The reaction was carried out according to the method of Example 32 of WO2023102085A1.

2 g of compound Va, 1.12 g of cyclopropanecarboxamide and 3.4 g of K₂CO₃ were added to a solution of 9.2 mL of acetonitrile and 15.2 mL of toluene, and the mixture was purged with N₂. Then, 36 mg of Pd(OAc)₂ and 62 mg of (R)-N,N-dimethyl-1-((S)-2-diphenylphosphino)ferrocenyl)ethylamine were added, and the mixture was purged with N₂, heated to 75 °C, stirred at that temperature. The reaction was not complete, and additional 36 mg of Pd(OAc)₂ and 62 mg of (R)-N,N-dimethyl-1-((S)-2-diphenylphosphino)ferrocenyl)ethylamine were added, continued to react at 75 °C until completion. The resultant was cooled to 20 °C. 28 mL of a mixed solvent of 3:1 (v/v) water/saturated aqueous ammonium chloride solution was slowly added to quench the reaction. The mixture was filtered, and the filter cake was rinsed with water and dried at 50 °C to obtain 1.41 g of solid compound I. Yield 62.4%, purity 99.15%.

### Example 10: Preparation of compound I

9 g of compound Va, 2.44 g of cyclopropanecarboxamide, 8.26 g of K₃PO₄, 7.72 g of DIEA, 67 mg of Pd(OAc)₂ and 0.31 g of (R)-(-)-1-[(S)-2-(dicyclohexylphosphino)ferrocenyl]ethyldi-tert-butylphosphine were added to 90 mL of toluene. The mixture was heated to 110 °C and stirred at that temperature until completion of the reaction. Then, the mixture was cooled to 30 °C to 35 °C, 27 mL of water was added, the pH was adjusted to 7 with 2N HCl. And the mixture was stirred for 30 min, filtered and dried to obtain 9.16 g of compound I. Yield 90.1%, purity 98.18%.

### Example 11: Preparation of compound I

4.70 g of compound Va, 1.27 g of cyclopropanecarboxamide, 2.81 g of K₂CO₃, 4.74 g of DBU, 0.14 g of Pd(OAc)₂ and 0.13 g of (R)-N,N-dimethyl-1-((S)-2-diphenylphosphino)ferrocenyl)ethylamine were added to DME (105 ml, 15V). The mixture was heated to 80 °C and stirred at that temperature until completion of the reaction. Then, the mixture was cooled to 30 °C to 35 °C, filtered, and the filter cake was rinsed with DME, then was slurried with water, filtered and dried to obtain 4.44 g of compound I. Yield 83.7%, purity 98.18%.

### Example 12: Preparation of compound I

2 g of compound Va, 0.54 g of cyclopropanecarboxamide, 1.13 g of K₂CO₃, 1.76 g of DIEA, 30 mg of Pd(OAc)₂ and 0.12 g of (R)-N,N-dimethyl-1-((S)-2-diphenylphosphino)ferrocenyl)ethylamine were added to 20 mL of toluene. The mixture was heated to 110 °C and stirred at that temperature until completion of the reaction. Then the mixture was cooled to 30 °C to 35 °C, 30 mL of water was added and stirred, the pH was adjusted to about 7 with 2N HCl. The mixture was filtered, and the filter cake was rinsed with a small amount of toluene and dried to obtain 1.95 g of solid compound I. Yield 86.4%, purity 97.83%.

### Example 13: Preparation of compound I

2 g of compound Va, 540 mg of cyclopropanecarboxamide, 1.83 g of K₃PO₄, 2.02 g of DBU, 60 mg of Pd(OAc)₂ and 0.28 g of 4,5-bis(diphenylphosphino)-9,9-dimethylxanthene were added to 20 mL of toluene. The mixture was heated to 110 °C and stirred at that temperature until completion of the reaction. Then the mixture was cooled to room temperature, 20 mL of water was added, stirred for 30 min, filtered, and the filter cake was rinsed with toluene and dried to obtain 1.91 g of solid compound I. Yield 84.6%, purity 96.96%.

Although the present application has been described with reference to exemplary embodiments, it should be understood that the present application is not limited to the disclosed exemplary embodiments. Various modifications or changes may be made to the exemplary embodiments of the present specification without departing from the scope or spirit of the present application. The scope of the claims should be interpreted based on the broadest interpretation to cover all modifications and equivalent structures and functions.

## Claims

1. A preparation method of compound V, comprising the following steps: mixing deuterated methylamine hydrochloride with an inorganic base in a solvent, and then adding compound IV and an organic base to react to form compound V, as represented by the following reaction: 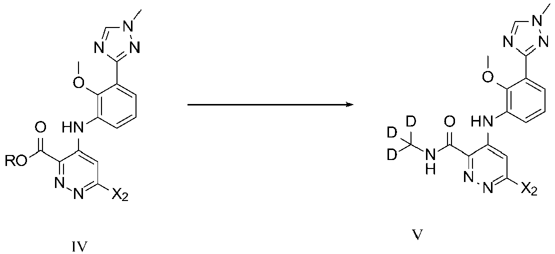 wherein,
R is C₁₋₆ alkyl or aryl, X₂ is selected from the group consisting of Cl, Br, I and sulfonate group, and the organic base is a metal ion-free non-nucleophilic organic base.

2. The preparation method according to claim 1, wherein R is selected from the group consisting of methyl and ethyl, preferably R is ethyl; X₂ is Cl.

3. The preparation method according to claim 1, wherein compound IV is compound IVa, compound V is compound Va, and compound IVa and compound Va are respectively as follows:

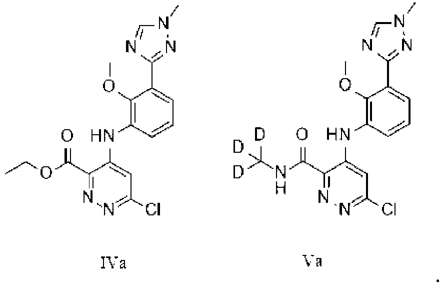

4. The preparation method according to any one of claims 1 to 3, wherein the inorganic base is optionally first dissolved in water to prepare an aqueous solution containing the inorganic base.

5. The preparation method according to any one of claims 1 to 4, wherein the inorganic base is selected from one or more of LiOH, NaOH, KOH, Ca(OH)₂, Ba(OH)₂, Na₂CO₃, K₂CO₃, NaHCO₃, KHCO₃, NaNH₂ and NH₃ ·H₂O, preferably one or more of NaOH and KOH.

6. The preparation method according to any one of claims 1 to 5, wherein the metal ion-free non-nucleophilic organic base is selected from one or more of DIEA, DBU, DBN, DMAP, 2,6-di-tert-butylpyridine, urea and thiourea, preferably one or more of DIEA, DBU, DMAP, urea and thiourea.

7. The preparation method according to any one of claims 1 to 6, wherein a molar ratio of compound IV to the metal ion-free non-nucleophilic organic base is 1:0.001 to 0.5; preferably 1:0.01 to 0.3; more preferably 1:0.05 to 0.15.

8. The preparation method according to any one of claims 1 to 7, wherein a molar ratio of compound IV to deuterated methylamine hydrochloride is 1:1 to 1.5, preferably 1:1 to 1.3.

9. The preparation method according to any one of claims 1 to 8, wherein a molar ratio of deuterated methylamine hydrochloride to the inorganic base is 1:1 to 1.5; preferably 1:1 to 1.2.

10. The preparation method according to any one of claims 1 to 9, wherein the solvent is selected from one or more of THF, DMF, methanol, ethanol, propanol, isopropanol, 1,4-dioxane and water; preferably one or more of ethanol, THF and water.

11. The preparation method according to any one of claims 1 to 10, further comprising the preparation of compound IV, as represented by the following reaction:
wherein R is C₁₋₆ alkyl or aryl, and X₁ and X₂ are independently selected from the group consisting of Cl, Br, I and sulfonate group.

12. The preparation method according to claim 11, wherein R is methyl or ethyl, preferably ethyl; X₁ and X₂ are independently Cl.

13. The preparation method according to any one of claims 11 to 12, wherein compound II is compound IIa, compound IV is compound IVa, and compound IIa and compound IVa are respectively as follows: .

14. The preparation method according to any one of claims 11 to 13, wherein compound II is reacted with compound III in a protic solvent in the presence of an organic base to form compound IV.

15. The preparation method according to claim 14, wherein the organic base is a metal ion-free non-nucleophilic organic base.

16. The preparation method according to claim 15, wherein the metal ion-free non-nucleophilic organic base is selected from the group consisting of DIEA, DBU, DBN, DMAP, 2,6-di-tert-butylpyridine, urea and thiourea, preferably one or more of DBU, DMAP, urea and thiourea, more preferably DBU or DIEA.

17. The preparation method according to any one of claims 15 to 16, wherein a molar ratio of compound III to the metal ion-free non-nucleophilic organic base is 1:1 to 3, preferably 1:2 to 3.

18. The preparation method according to any one of claims 14 to 17, wherein the protic solvent is an aliphatic alcohol containing 1 to 6 carbon atoms; preferably methanol, ethanol or isopropanol.

19. The preparation method according to any one of claims 11 to 18, wherein a molar ratio of compound III to compound II is 1:1 to 1.5, preferably 1:1 to 1.3.

20. The preparation method according to any one of claims 1 to 19, wherein compound V is further used to prepare compound I, and compound I is as follows: .

21. The preparation method according to claim 20, wherein compound V is reacted with cyclopropanecarboxamide in a non-polar organic solvent in the presence of a base, a palladium catalyst and a phosphine ligand to form compound I, as represented by the following reaction:
wherein X₂ is selected from the group consisting of Cl, Br, I and sulfonate group, preferably X₂ is Cl.

22. The preparation method according to claim 21, wherein the base is selected from the group consisting of an inorganic base, an organic base and a mixture thereof; preferably a mixture of an inorganic base and an organic base.

23. The preparation method according to claim 22, wherein the inorganic base is selected from one or more of LiOH, NaOH, KOH, Ca(OH)₂, Ba(OH)₂ , Na₂CO₃, K₂CO₃, NaHCO₃, KHCO₃, NaNH₂, NH₃ ·H₂O, K₃PO₃ and K₂HPO₃; preferably K₂CO₃ or K₃PO₃.

24. The preparation method according to any one of claims 22 to 23, wherein a molar ratio of compound V to the inorganic base is 1:1 to 3; preferably, a molar ratio of compound V to the inorganic base is 1:1.5 to 2.5.

25. The preparation method according to claim 22, wherein the organic base is a metal ion-free non-nucleophilic organic base.

26. The preparation method according to claim 25, wherein the metal ion-free non-nucleophilic organic base is selected from the group consisting of DIEA, DBU, DBN, DMAP, 2,6-di-tert-butylpyridine, urea and thiourea, preferably one or more of DIEA, DBU, DMAP, urea and thiourea, more preferably DBU or DIEA.

27. The preparation method according to any one of claims 25 to 26, wherein a molar ratio of compound V to the metal ion-free non-nucleophilic organic base is 1:1 to 5; preferably, a molar ratio of compound V to the metal ion-free non-nucleophilic organic base is 1:2 to 3.

28. The preparation method according to any one of claims 21 to 27, wherein the palladium catalyst is selected from the group consisting of Pd(OAc)₂, PdCl₂(MeCN)₂, Pd₂(dba)₃, Pd(dba)₂, [(allyl)PdCl]₂ and [(crotyl)PdCl]₂; preferably Pd(OAc)₂.

29. The preparation method according to any one of claims 21 to 28, wherein a molar ratio of compound V to the palladium catalyst is 1:0.005 to 0.1, preferably 1:0.01 to 0.05.

30. The preparation method according to any one of claims 21 to 29, wherein the phosphine ligand is selected from the group consisting of a Josiphos ligand and a Xantphos ligand.

31. The preparation method according to claim 30, wherein the Josiphos ligand is selected from the group consisting of (R)-N,N-dimethyl-1-((S)-2-diphenylphosphino)ferrocenyl)ethylamine and (R)-(-)-1-[(S)-2-(dicyclohexylphosphino)ferrocenyl]ethyldi-tert-butylphosphine; and the Xantphos ligand is 4,5-bis(diphenylphosphino)-9,9-dimethylxanthene.

32. The preparation method according to any one of claims 21 to 31, wherein a molar ratio of compound V to the phosphine ligand is 1:0.01 to 0.1, preferably 1:0.02 to 0.05.

33. The preparation method according to any one of claims 21 to 32, wherein the non-polar organic solvent is a single solvent selected from the group consisting of an ether solvent, an aromatic solvent and an ester solvent; preferably DME or toluene.

34. A preparation method of compound IV, comprising the following steps: reacting compound II with compound III in a protic solvent in the presence of an organic base to form compound IV, as represented by the following reaction:
wherein R is C₁₋₆ alkyl or aryl, X₁ and X₂ are independently selected from the group consisting of Cl, Br, I and sulfonate group, and the organic base is a metal ion-free non-nucleophilic organic base.

35. The preparation method according to claim 34, wherein R is methyl or ethyl, preferably ethyl.

36. The preparation method according to any one of claims 34 to 35, wherein X₁ and X₂ are independently Cl.

37. The preparation method according to any one of claims 34 to 36, wherein compound II is compound IIa, compound IV is compound IVa, and compound IIa and compound IVa are respectively as follows: .

38. The preparation method according to any one of claims 34 to 37, wherein the protic solvent is an aliphatic alcohol containing 1 to 6 carbon atoms; preferably methanol, ethanol or isopropanol, more preferably ethanol.

39. The preparation method according to any one of claims 34 to 38, wherein the metal ion-free non-nucleophilic organic base is selected from the group consisting of DIEA, DBU, DBN, DMAP, 2,6-di-tert-butylpyridine, urea and thiourea, preferably one or more of DBU, DMAP, urea and thiourea, more preferably DBU or DIEA.

40. The preparation method according to any one of claims 34 to 39, wherein a molar ratio of compound III to compound II is 1:1 to 1.5, preferably 1:1 to 1.3.

41. The preparation method according to any one of claims 34 to 40, wherein a molar ratio of compound III to the metal ion-free non-nucleophilic organic base is 1:1 to 3, preferably 1:2 to 3.

42. The preparation method according to any one of claims 34 to 41, wherein a mass-to-volume ratio of compound III to the protic solvent is 1:5 to 20, preferably 1:8 to 15.

43. The preparation method according to any one of claims 34 to 42, wherein a temperature for a reaction of compound II with compound III is 60 °C to 85 °C; preferably 65 °C to 80 °C.

44. A preparation method of compound I, comprising the following steps: reacting compound V with cyclopropanecarboxamide in a non-polar organic solvent in the presence of a base, a palladium catalyst and a phosphine ligand, as represented by the following reaction:
wherein X₂ is independently selected from the group consisting of Cl, Br, I and sulfonate group.

45. The preparation method according to claim 44, wherein X₂ is Cl.

46. The preparation method according to any one of claims 44 to 45, wherein the non-polar organic solvent is a single solvent.

47. The preparation method according to any one of claims 44 to 46, wherein the base is selected from the group consisting of an inorganic base, an organic base and a mixture thereof; preferably, the base is selected from the group consisting of a mixture of an inorganic base and an organic base.

48. The preparation method according to claim 47, wherein the inorganic base is selected from one or more of LiOH, NaOH, KOH, Ca(OH)₂, Ba(OH)₂, Na₂CO₃, K₂CO₃, NaHCO₃, KHCO₃, NaNH₂, NH₃ ·H₂O, K₃PO₃ and K₂HPO₃ preferably, the inorganic base is selected from the group consisting of K₂CO₃ and K₃PO₃.

49. The preparation method according to any one of claims 47 to 48, wherein a molar ratio of compound V to the inorganic base is 1:1 to 3; preferably 1:1.5 to 2.5.

50. The preparation method according to claim 47, wherein the organic base is a metal ion-free non-nucleophilic organic base.

51. The preparation method according to claim 50, wherein the metal ion-free non-nucleophilic organic base is selected from one or more of DIEA, DBU, DBN, DMAP, 2,6-di-tert-butylpyridine, urea and thiourea; preferably, the metal ion-free non-nucleophilic organic base is selected from one or more of DBU, DIEA, DMAP, urea and thiourea; more preferably, the metal ion-free non-nucleophilic organic base is selected from the group consisting of DBU and DIEA.

52. The preparation method according to any one of claims 50 to 51, wherein a molar ratio of compound V to the metal ion-free non-nucleophilic organic base is 1:1 to 5; preferably 1:2 to 3.

53. The preparation method according to any one of claims 44 to 52, wherein the palladium catalyst is selected from the group consisting of Pd(OAc)₂, PdCl₂(MeCN)₂, Pd₂(dba)₃, Pd(dba)₂, [(allyl)PdCl]₂ and [(crotyl)PdCl]₂; preferably, the palladium catalyst is Pd(OAc)₂.

54. The preparation method according to any one of claims 44 to 53, wherein a molar ratio of compound V to the palladium catalyst is 1:0.005 to 0.1; preferably 1:0.01 to 0.05.

55. The preparation method according to any one of claims 44 to 54, wherein the phosphine ligand is selected from the group consisting of a Josiphos ligand and a Xantphos ligand.

56. The preparation method according to claim 55, wherein the Josiphos ligand is selected from the group consisting of (R)-N,N-dimethyl-1-((S)-2-diphenylphosphino)ferrocenyl)ethylamine and (R)-(-)-1-[(S)-2-(dicyclohexylphosphino)ferrocenyl]ethyldi-tert-butylphosphine; and the Xantphos ligand is 4,5-bis(diphenylphosphino)-9,9-dimethylxanthene.

57. The preparation method according to any one of claims 44 to 56, wherein a molar ratio of compound V to the phosphine ligand is 1:0.01 to 0.1; preferably 1:0.02 to 0.05.
